# EUROPÄISCHE PATENTSCHRIFT

(11) **EP 0 987 047 B1**
(45) Veröffentlichungstag und Bekanntmachung des Hinweises auf die Patenterteilung: **06.04.2005**
(21) Anmeldenummer: 99250310.2
(22) Anmeldetag: 03.09.1999
(51) Int. Cl.: A61N 1/37, A61B 1/00

(54) **Anordnung zur Patientenüberwachung**
Patient monitoring device
Dispositif de surveillance d'un patient

(30) Priorität: 18.09.1998 DE 19844296
(43) Veröffentlichungstag der Anmeldung: 22.03.2000
(73) Patentinhaber: BIOTRONIK Mess- und Therapiegeräte GmbH & Co Ingenieurbüro Berlin, 12359 Berlin (DE)
(72) Erfinder: Lang, Bernhard, 90537 Feucht (DE); Bolz, Armin, 76356 Weingarten (DE); Neudecker, Johannes, D-91054 Erlangen (DE)
(74) Vertreter: Eisenführ, Speiser & Partner

(56) Entgegenhaltungen:
- WO-A-97/00708
- DE-A- 4 441 907
- US-A- 4 675 656
- US-A- 5 204 670
- US-A- 5 720 770
- US-A- 5 782 878

## Beschreibung

Die Erfindung betrifft eine Anordnung zur Patientenüberwachung gemäß dem Oberbegriff des Anspruchs 1.

Bei schwerwiegenden Gesundheitsstörungen bzw. nach größeren medizinischen Eingriffen, so zum Beispiel nach einer Herztransplantation zur frühzeitigen Erkennung einer etwaigen Abstoßungsreaktion, ist eine längerwährende ununterbrochene Überwachung des Zustandes des Patienten angezeigt. Mit Blick auf die Lebensqualität des Betroffenen, aber auch aus Kapazitäts- und Kostengründen sollte diese außerhalb einer Klinik erfolgen.
Auch bei Trägern implantierter medizinelektronischer Geräte, etwa von Herzschrittmachern, ist in manchen Fällen die ständige Überwachung des Zustandes des Patienten bzw. des Gerätes erforderlich und in aller Regel zumindest die Möglichkeit einer sofortigen Signalisierung von lebensbedrohlichen Zuständen des Patienten oder des Gerätes, verbunden mit der gleichzeitigen Bestimmung des Aufenthaltsortes des Patienten, wünschenswert.

Für verschiedene Anwendungen gibt es eine Vielzahl bekannter Anordnungen zur nicht-stationären Patientenüberwachung.

In US 5 626 630 ist ein mit einem implantierbaren quasi-passiven Transponder arbeitendes medizinisches Telemetriesystem beschrieben, das neben dem Transponder ein am Körper des Patienten zu tragendes Relaisgerät und eine entfernte Überwachungsstation umfaßt.

In der deutschen Patentanmeldung P 197 58 393.8 der Anmelderin wird eine Anordnung zur Patientenüberwachung beschrieben, bei der ein Patientengerät selbsttätig im Ansprechen auf eine bestimmte Position des Patienten zur Datenübertragung an eine zentrale Überwachungsstelle, insbesondere über ein Telefonnetz, aktiviert wird.

WO 97/00708 beschreibt ein fortgeschrittenes, sehr aufwendiges System zur weltweiten Patientenlokalisierung und zur Datenübertragung von implantierten Geräten zu geeigneten Auswertungspunkten. Zur Bestimmung der geografischen Position des Patienten aufgrund des Satellitennavigationssystems GPS umfaßt das System einen speziellen Empfänger, den der Patient mit sich führt.

Auf die gleiche aufwendige Weise erfolgt die Bestimmung der Patientenposition bei dem Herzstimulationssystem mit erweiterten Kommunikations- und Überwachungsmöglichkeiten gemäß der US 5 720 770, die im übrigen ebenfalls die Nutzung des Telefon-Festnetzes oder eines Mobilfunknetzes für die Übertragung relevanter Daten vorsieht.

Der Erfindung liegt die Aufgabe zugrunde, eine einfache und kostengünstige Anordnung zur Patientenüberwachung, insbesondere von Trägern implantierter medizinelektronischer Geräte, anzugeben, die eine hinreichend genaue Bestimmung des Aufenthaltsortes des Patienten in einem Notfall erlaubt.

Die Aufgabe wird durch eine Anordnung mit den im Anspruch 1 angegebenen Merkmalen gelöst.
Die Erfindung schließt den grundlegenden Gedanken ein, zur Positionsermittlung des Patienten die in einem zellulären Mobilfunknetz ständig intern verfügbaren positionsrelevanten Informationen zu nutzen und auf gesonderte Mittel zur geografischen Positionsbestimmung zu verzichten.

Dieser Gedanke beruht zum einen darauf, daß beim Betreiber eines Mobilfunknetzes die geografischen Koordinaten allereingesetzten Basisstationen vorliegen und jedes angemeldete Endgerät sich natürlich im Sende- und Empfangsbereich mindestens einer Basisstation, normalerweise aber mehrerer Basisstationen gleichzeitig, befindet. Auf der Vermittlungsebene des Mobilfunksystems liegt auch die Information darüber vor, um welche Basisstation es sich dabei handelt, so daß eine näherungsweise Ortung des Patienten schon auf der Grundlage des Basisstations-Positionsdatensatzes möglich ist.

Weiterhin erfolgen in einem modernen Mobilfunksystem zur Realisierung der automatischen Verbindungsübergabe zwischen verschiedenen Basisstationen ("Handover") unter anderem Messungen der Signallaufzeit mindestens zur aktuell für das betreffende Endgerät aktiven Basisstation, so daß im System mit der Laufzeitinformation eine weitere positionsrelevante Information vorliegt. In dem Fall, daß zugleich die Laufzeiten zu mehreren benachbarten Basisstaionen gemessen werden, läßt sich aus deren Positionsdaten und den zugehörigen Laufzeitwerten eine sehr präzise Bestimmung des Ortes des betreffenden Endgerätes und somit des Patienten gewinnen. Die Laufzeitdaten können, je nach konkreter Organisation des Netzes, an den Endgeräten oder bei den Basisstationen und ggfs. auch auf der Vermittlungsebene abgefragt werden.

Schließlich bietet grundsätzlich auch der Umstand, daß die Basisstationen in der Regel mit Richtantennen arbeiten, eine Möglichkeit zur verfeinerten Positionsbestimmung des Patienten, indem bestimmt und ausgewertet wird, mit welcher Antenne der Basisstation die Verbindung mit dem Patienten-Endgerät gehalten wird.

Ausgehend von diesen Überlegungen ist es sinnvoll, daß die Überwachungszentrale des Patientenbetreungssystems zum Empfang von Positionsdaten des Mobilfunk-Endgerätes (neben der Verbindung mit dem Patienten-Endgerät) mit einer Mobilfunk-Vermittlungsstelle oder der Zentrale des Mobilfunknetzes verbunden ist.

Der Überwachungszentrale sind Positionsdaten-Auswertungsmittel zur Auswertung von die Entfernung des Mobilfunk-Endgerätes zu mindestens einer Basisstation widerspiegelnden Laufzeitdaten zusammen mit Koordinatendaten der aktiven bzw. erreichbaren Basisstation(en) des Mobilfunknetzes zugeordnet - was die Variante einschließt, daß diese Auswertungsmittel im Verantwortungsbereich des Mobilfunknetzbetreibers liegen und lediglich die Ergebnis-Daten in das Patientenüberwachungssystem eingespeist werden.

Aus obigen grundsätzlichen Gedanken ist klar, daß die gleichzeitige Verarbeitung von Laufzeitdaten bezüglich mehrerer Basisstationen bevorzugt ist. Eine Bestimmung der Signallaufzeiten zu mehreren Basisstationen kann, sofern sie nicht im Standardprotokoll des benutzten Netzes ohnehin vorgesehen ist, im Rahmen eines speziellen Protokolls organisiert sein, das zwischen dem Betreiber des Patientenüberwachungssystems und dem Betreiber des Mobilfunknetzes vereinbart wird. Die Auswertung mittels bekannter trigonometrischer Ortungsverfahren erfolgt am zweckmäßigsten bei der Patientenüberwachungszentrale.

Eine weitere bevorzugte Ausführung besteht darin, daß die Positionsbestimmungseinheit Laufzeit-Speichermittel zur Speicherung der Zeitabhängigkeit von Laufzeitdaten über einen vorbestimmten Zeitraum aufweisen. Auf diese Weise kann ggfs. eine Bewegungsbahn des Patienten innerhalb des Sendebereiches der aktuellen Basisstation (oder mehrere Basisstationen) rekonstruiert werden, die für die genaue Bestimmung der aktuellen Position hilfreich ist. Hierfür kann es insbesondere sinnvoll sein, den Zeitraum soweit zurückreichend zu wählen, daß frühere Verbindungen zu anderen Basisstationen mitvernünftiger Wahrscheinlichkeit erfaßt werden.
Nach obigem besteht eine weitere bevorzugte Ausführung darin, daß die Positionsbestimmungseinheit Mittel zur Erfassung der Antennenzuordnung bei der Verbindung des Mobilfunk-Endgerätes mit der aktiven Basisstation aufweisen.

Für die Ausführung der Erfindung ist zweckmäßigerweise ein gut ausgebautes GSM-Mobilfunknetz zu wählen, nach derzeitigem Stand insbesondere ein D-Netz oder das E-Netz. Im Interesse einer global möglichst lückenarmen Patientenüberwachung besonders vorteilhaft ist eine Anbindung des Patientenüberwachungssystems an sämtliche etablierte Mobilfunknetze mit hohem Flächendeckungsgrad.

Um den Leistungsbedarf der Patienteneinheit (des Mobilfunk-Endgerätes) und damit den Wartungsaufwand niedrig zu halten und letztlich auch einen hohen Miniaturisierungsgrad zu ermöglichen, sollte eine Aktivierung nur im Bedarfsfall oder allenfalls zusätzlich zu Überprüfungszwecken in größeren Abständen erfolgen.
In vorteilhafter Weise sind daher eine mit der Körpersignal-Verarbeitungseinheit oder dem Therapiegerät verbundene Parameterüberwachungseinheit zur Überwachung der Werte des physiologischen Parameters bzw. eines Betriebsparameters des Therapiegerätes und mit dem Ausgang der Parameterüberwachungseinheit verbundene Schaltmittel zur Aktivierung des Mobilfunk-Endgerätes im Ansprechen auf die Erfassung eines anormalen Wertes vorgesehen. Weiterhin können manuell oder akustisch betätigte Schaltmittel zur Aktivierung des Endgerätes durch den Patienten selbst vorgesehen sein.

Eine besonders nützliche Anwendung der Erfindung wird bei einem implantierten Herztherapiegerät - etwa einem Herzschrittmacher zur antibradykarden oder antitachykarden Stimulation des Herzens oder einem automatischen Defibrillator oder Kardioverter - mit einer angeschlossenen Reiz- bzw. Schockelektrodenanordnung gesehen, bei dem der Körperfühler und die nachgeschaltete Körpersignal-Verarbeitungseinheit zur Aufnahme und Verarbeitung von Herzsignalen zur Erfassung von, insbesondere lebensbedrohlichen, Herzrhythmusstörungen (z.B: ventrikulären Fibrillationen oder Vorstufen davon) ausgebildet sind.

Die Körpersignal-Verarbeitungseinheit umfaßt hierbei insbesondere eine Ratenbestimmungs- und -auswertungseinheit zur Bestimmung und vorzugsweise auch retrospektiven Auswertung der Herzrate als physiologischem Parameter und die Parameterüberwachungseinheit Speicher- und Vergleichermittel zur Speicherung mindestens eines auf die Herzrate bzw. deren Zeitabhängigkeit bezogenen Grenzwertes und zum Vergleich mit diesem Grenzwert zur Ausgabe eines Steuersignals an die Schaltmittel bei Überschreitung des Grenzwertes.

Weiterhin umfaßt die Parameterüberwachungseinheit zweckmäßigerweise Erfassungs-, Speicher- und Vergleichermittel zur Überwachung mindestens der Batteriespannung und/oder der Impedanz der Reiz- bzw. Schockelektrodenanordnung und/oder der Reizimpulsamplitude zur Ausgabe eines Steuersignals an die Schaltmittel bei Unterschreitung eines Batteriespannungs- oder Impulsamplituden-Grenzwertes bzw. bei Überschreitung eines Elektrodenimpedanz-Grenzwertes.

Das Mobilfunk-Endgerät - das im übrigen und bei Vorsehen der oben angesprochenen speziellen Mittel zur Notfallaktivierung durchaus ein normales Mobiltelefon sein kann - weist eine Schnittstelleneinrichtung zur Verbindung mit der Körpersignal-Verarbeitungseinheit und/oder dem Therapiegerät auf, die Codierungsmittel zur Umsetzung von Körpersignal- bzw. Therapiegerätedaten in das Datenformat des Mobilfunknetzes umfaßt.

Vorteilhafte Weiterbildungen der Erfindung sind im übrigen in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine Prinzipskizze einer bevorzugtes Ausführungsform der Gesamtanordnung,
- Figur 2: ein vereinfachtes Funktions-Blockschaltbild der patientenseitigen Komponenten einer modifizierten Anordnung nach Fig. 1 und
- Figur 3: ein vereinfachtes Funktions-Blockschaltbild der Mittel zur Positionsbestimmung bei einer weiteren modifizierten Ausführungsform.

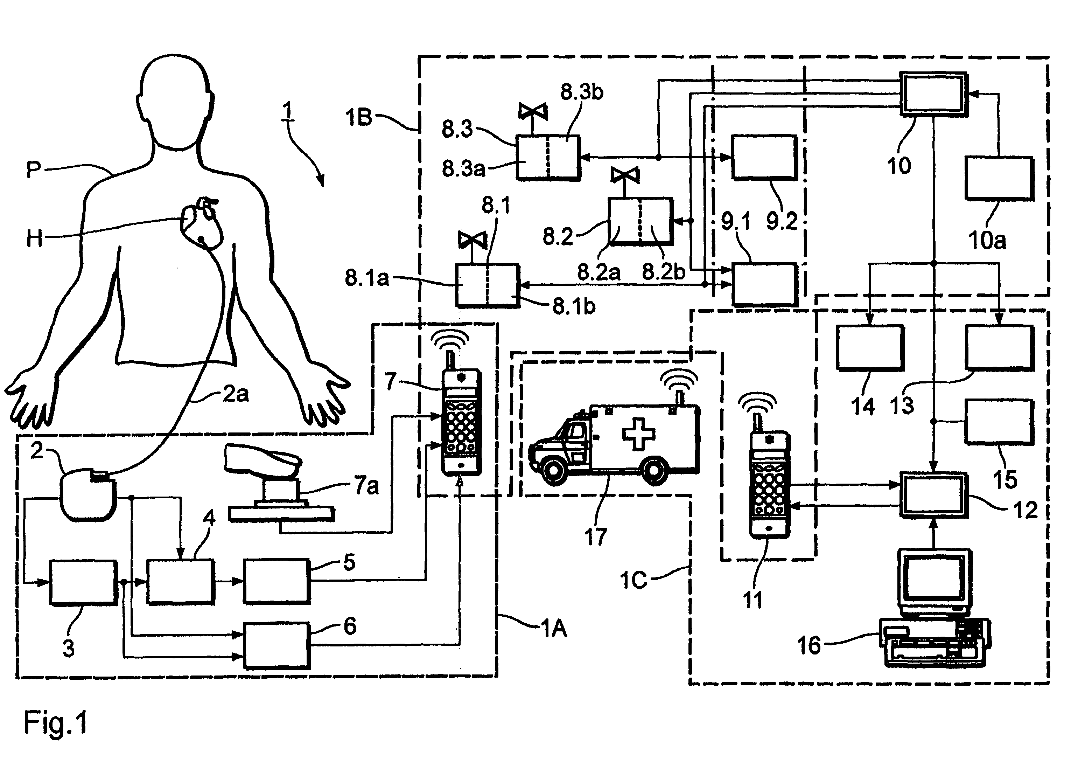

In Fig. 1 ist eine Patientenüberwachungsanordnung 1 zur Überwachung und Feststellung des Aufenthaltsortes eines Schrittmacherpatienten P gezeigt.

Der Patient P hat einen Herzschrittmacher 2 mit einer zum Herzen H geführten Elektrodenleitung 2a, die den implantierten Teil der Anordnung 1 bilden. Die Elektrodenleitung 2a ist zugleich ein Fühler für die Herzaktivität als physiologischer Parameter und Stimulationselektrode. Über eine als solche bekannte (in der Figur nicht dargestellte) Telemetrieverbindung ist der implantierte Schrittmacher 2 mit einer Körpersignal-Verarbeitungseinheit 3 und sowohl direkt als auch - über einen zweiten Datenpfad - über die Körpersignal-Verarbeitungseinheit mit einer Parameterüberwachungseinheit 4 verbunden. Dem Ausgang der Parameterüberwachungseinheit 4 ist eine Schalteinrichtung 5 nachgeordnet. Weiterhin sind der Schrittmacher 2 und die Körpersignal-Verarbeitungseinheit 3 mit einer Schnittstelleneinrichtung 6 verbunden. Die Schnittstelleneinrichtung 6 ist über eine Datenverbindung und die Schalteinheit 6 über eine Steuersignalverbindung mit einem Mobiltelefon 7 verbunden. Diesem ist außerdem ein manuell zu betätigender Einschalter 7a zugeordnet (der in der Praxis durch eine entsprechende Funktionstaste des Mobiltelefons selbst gebildet sein wird). Die vorgenannten Komponenten bilden eine Patienteneinheit 1A.

Das Mobiltelefon 7 ist zugleich Element eines GSM-Mobilfunknetzes 1 B, zu dem weiterhin eine Mehrzahl von Basisstationen gehört, von denen in der Figur drei benachbarte Basisstationen 8.1 bis 8.3 gezeigt sind. Diese umfassen jeweils eine Basis-Sendestation (BTS = Base Transceiver Station) 8.1 a, 8.2a bzw. 8.3a sowie einen Basisstations-Controller (BSC = Base Station Controller) 8.1b, 8.2b bzw. 8.3b. Auf der Ebene des Vermittlungs-Subsystems sind im dargestellten Beispiel den Basisstationen 8.1 und 8.2 ein und dasselbe Mobilschaltzentrum (MSC = Mobile Switching Centre) 9.1 zugeordnet, und der Basisstation 8.3 ist ein anderes MSC 9.2 zugeordnet. Allen drei Basisstationen 8.1 bis 8.3 ist ein Betriebs- und Wartungszentrum (OMC = Operation and Maintenance Centre) 10 zugeordnet. Die Funktionsverteilung zwischen den BTS/PSC, den MSC und dem OMC ist im jeweiligen System konkret definiert und bedarf hier keiner allgemeinen Erläuterung.

Hierwird lediglich vorausgesetzt, daßdem OMC 10 ein Basisstationen-Koordinatenspeicher 10a zugeordnet ist, in dem die geografischen Positionsdaten aller Basisstationen des Netzes 1 B gespeichert sind, und daß der OMC zur zentralen Registrierung der gemessenen Laufzeiten und der erfaßten Diversity-Zuordnungen bei den Basisstationen ausgebildet ist. Schließlich gehört zum Netz 1B auch ein zweites in der Figur gezeigtes Mobiltelefon, nämlich ein einer Patientenüberwachungszentrale 1 C zugeordnetes Endgerät 11.

Kernstück der Patientenüberwachungszentrale 1C ist ein Zentralrechner 12, der bidirektional mit dem Endgerät 11 (das hier symbolisch für eine in der Praxis erforderliche Mehrzahl von Endgeräten steht) und zudem eingangsseitig mit dem OMC 10 verbunden ist. Dem Zentralrechner 12 sind zudem ein Koordinaten-Pufferspeicher 13, ein Laufzeit-Pufferspeicher 14 für die gemessenen Laufzeitwerte, ein Antennenzuordnungs-Pufferspeicher 15 für die erfaßten Antennenzuordnungen der Basisstation(en) bezüglich des Endgerätes 7 und eine Mehrzahl von PC-Arbeitsplätzen zugeordnet, für die wieder symbolisch lediglich ein PC 16 dargestellt ist. Der Patientenüberwachungszentrale sind schließlich Notfall-Einsatzkräfte zugeordnet, die in der Figur durch das Krankenfahrzeug 17 symbolisiert sind und ebenfalls - was im Interesse der Übersichtlichkeit allerdings nicht im einzelnen dargestellt ist - über das Mobilfunknetz 1 B zum Einsatz gebracht werden können.

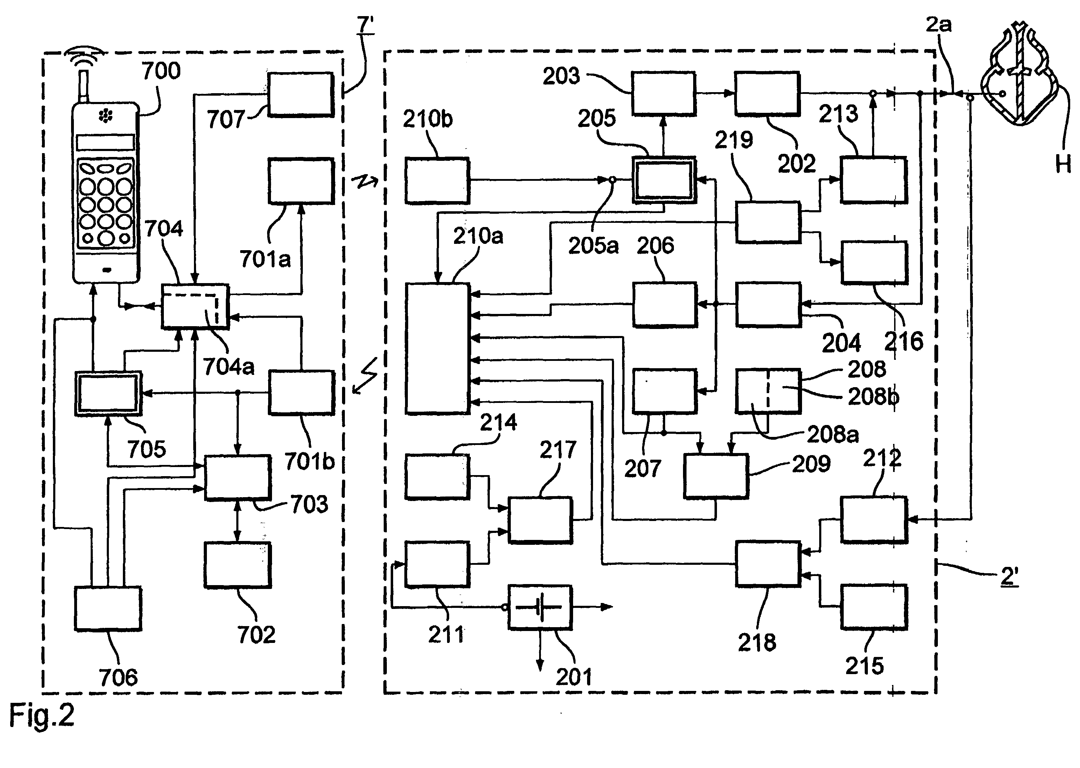

Fig. 2 ist ein vereinfachtes Funktions-Blockschaltbild des gegenüber Fig. 1 modifizierten patientenseitigen Teils 1 A' einer Patientenüberwachungsanordnung, das nur die für die Erläuterung der Ausführung der Erfindung wesentlichen Funktionskomponenten zeigt. Im Unterschied zur Prinzipdarstellung in Fig. 1 sind die der Körpersignal-Verarbeitungseinheit sowie der Parameterüberwachungseinheit zuzurechnenden Funktionseinheiten hier in den Schrittmacher selbst integriert.

Der Herzschrittmacher 200 hat im übrigen einen weitgehend bekannten Aufbau und ist von einem Typ, der zumindest eine Ventrikelstimulation sowie die Erfassung ventrikulärer Herzsignale ermöglicht. Hierzu umfaßt er eine Batterie 201 zur Stromversorgung, einen über eine Ausgangsstufe 202 ausgangsseitig mit der im Ventrikel des Herzens H plazierten Ventrikelelektrode 2a verbundenen Stimulationsimpulsgenerator 203 sowie eine eingangsseitig mit der Elektrode 2a verbundene Eingangsstufe 204. Die Schrittmachersteuerfunktionen sind in der Figur im Steuerblock 205 zusammengefaßt, der Programmierungseingänge 205a zur externen Programmierung der Impulsrate und -amplitude sowie zur Aktivierung von Zusatzfunktionen, beispielsweise von Tachykardieterminierungs-Impulsfolgen, hat.

Der Eingangsstufe 204 sind (neben dem Steuerblock 205) ein Herzsignal-Zwischenspeicher 206 und eine Herzratenbestimmungsstufe 207 nachgeschaltet, in der aufgenommene Herzsignale (IEGM = intracardial electrograms) zwischengespeichert und zudem zur Bestimmung der aktuellen Herzrate verarbeitet werden. Weiterhin umfaßt der Schrittmacher einen Ratengrenzwertspeicher 208 mit zwei Speicherbereichen 208a und 208b für einen oberen bzw. einen unteren Ratengrenzwert und eine eingangsseitig mit dem Speicher 208 sowie mit der Herzratenbestimmungsstufe verbundene Ratenvergleichereinheit 209. Ausgangsseitig sind sowohl der Herzsignal-Zwischenspeicher 206 als auch die Herzratenbestimmungsstufe 207 sowie die Ratenvergleichereinheit 209 mit einer schrittmacherseitigen Telemetrie-Sendeeinheit 210a verbunden.

Der Schrittmacherbatterie 201 ist in an sich bekannter Weise eine Batteriezustands-Erfassungseinheit 211 zugeordnet, und der Ausgangsstufe 202 sind eine Elektrodenimpedanz-Erfassungseinheit 212 und eine Impulsamplituden-Erfassungseinheit 213 zugeordnet, die ebenfalls an sich bekannt sind. Den Detektoreinheiten 211 bis 213 sind jeweils Grenzwertspeicher 214 für die Batteriespannung, 215 für die Elektrodenimpedanz und 216 für die Impulsamplitude und jeweils eine Vergleichereinheit 217 bis 219 zu- bzw. nachgeordnet. Die Ausgänge der Vergleichereinheiten 217 bis 219 sind mit der Telemetrie-Sendeeinheit 210a verbunden. Eine schrittmacherseitige Telemetrie-Empfangseinheit 210b ist mit den Programmiereingängen 205a des Steuerblocks 205 verbunden. Die schrittmacherseitigen Telemetrieeinheiten 210a, 210b sind - ebenso wie die externe Einheiten (siehe weiter unten) für langreichweitige Telemetrie mit einer Reichweite von 1 bis 2 Metern ausgelegt.

Ein externes Patientengerät 7' umfaßt ein weitgehend herkömmlich aufgebautes Mobiltelefon 700 und eine (zusammen mit der implantierten Sende- und Empfangseinheit 210b und 210a) eine bidirektionale Telemetriestrecke zum Herzschrittmacher realisierende Sende- und Empfangseinheit 701a, 701b. Es umfaßt weiterhin einen Pufferspeicher 702 mit diesem zugeordneter Speicherzugriffssteuerung 703 zur Zwischenspeicherung von über die Telemetriestrecke empfangenen Daten und eine das Übertragungsprotokoll des jeweiligen Mobilfunknetzes unterstützende Schnittstelle (etwa PCMCIA-Karte) 704, die eine Codierungseinheit 704a aufweist, zur Verbindung mit dem Mobiltelefonteil 700 und zur Umsetzung der Datenformate der an die Patientenüberwachungszentrale 1C (Fig. 1) zu übermittelnden Daten und der von dieser erhaltenen Programmierungsdaten.

Schließlich umfaßt das Patientengerät eine Steuerstufe 705 zur selbsttätigen Steuerung eines Notrufes, die eingangsseitig mit der Telemetrie-Empfangseinheit 701b, ausgangsseitig mit dem Telefonteil 700 und der Schnittstelle 704 sowie einund ausgangsseitig mit der Speicherzugriffssteuerung 703 verbunden ist. Schließlich ist eine manuell zu betätigende Notwahl-Schalteinheit 706 zur manuellen Auslösung eines Notrufes vorgesehen, die ebenfalls mit dem Telefonteil, der Speicherzugriffssteuerung und der Schnittstelle verbunden ist. Ein mit der Schnittstelle 704 verbundener Identifikationsdatenspeicher 707 enthält invariable Geräte- und Patientenidentifikationsdaten.

Nachfolgend werden die für die Erfindung relevanten Aspekte der Funktionsweise der Ausführung gemäß den Figuren 1 bis 3 erläutert; Einzelheiten der Schrittmacherfunktionen (einschließlich der Telemetriefunktion) sowie der Datenübertragung in einem Mobilfunknetz sind als aus dem Stand der Technik bekannt vorauszusetzen. Im weiteren wird auch vorausgesetzt, daß die Mobilfunkstrecke neben den Mitteln zur Datenübertragung in üblicher Weise auch einen Sprachkanal aufweist.

Im laufenden Betrieb des Schrittmachers 2 bzw. 2' werden permanent durch die Körpersignal-Verarbeitungseinheit 3 bzw. - im konkreten Beispiel der Fig. 2 - durch die Herzratenbestimmungsstufe 207, den Ratengrenzwertspeicher 208 und die Ratenvergleichereinheit 209 die Herzrate sowie durch die Parameterüberwachungseinheit 4 die Funktion des Schrittmachers - gemäß Fig. 2 durch die Stufen 211 bis 219 konkret hinsichtlich der Batteriespannung, Impulsamplitude und Elektrodenimpedanz - überwacht. Wird im Ergebnis eines der Grenzwert-Vergleiche festgestellt, daß ein relevanter Meßwert den zulässigen Bereich verlassen hat, wird durch das diesen Umstand kennzeichnende Signal in der Ausführung nach Fig. 1 durch die Schalteinrichtung 5 unmittelbar das Mobiltelefon 7 aktiviert. Der Ablauf umfaßt dabei die automatische Einschaltung des Telefons, die Wahl einer (in einem internen, nicht gesondert dargestellten Speicher des Telefonteils) vorgespeicherten Notrufnummer und die Absetzung eines im Schrittmacher 2 gespeicherten Datenstrings über die Schnittstelle 6 nach erfolgtem Verbindungsaufbau.

In der modifizierten Ausführung nach Fig. 2 wird durch ein Ausgangssignal einer der Vergleichereinheiten 209, 217, 218 oder 219, das einen unzulässigen Wert einer der überwachten Größen widerspiegelt, selbsttätig die Telemetrie-Sendeeinheit 210a aktiviert. Diese übermittelt daraufhin unter Steuerung durch den Steuerblock 205 ein Aktivierungssignal sowie einen vorbestimmten (primären) Datenstring, der speziell das aus dem Herzsignalspeicher 206 ausgelesene IEGM und den am Ausgang der Herzratenbestimmungsstufe 207 verfügbaren aktuellen Wert der Herzrate umfaßt, an das externe Patientengerät 7'.

Dort werden das Aktivierungssignal und der Datenstring durch die externe Telemetrie-Empfangseinheit 701 b empfangen und der externen Steuerstufe 705 zugeführt, und der primäre Datenstring wird - unter Steuerung durch die Steuerstufe sowie die Speicherzugriffssteuerung 703 - zunächst im externen Pufferspeicher abgelegt. Nach Einschaltung des Mobiltelefons 700 und erfolgtem Verbindungsaufbau zum angerufenen Endgerät 11 der Patientenüberwachungszentrale 1C wird der primäre Datenstring aus dem Pufferspeicher ausgelesen und mit Identifikationsdaten aus dem Identifikationsdatenspeicher 707 zu einem sekundären Datenstring ergänzt, der in der Codierungseinheit 704a codiert und als Notruf an die Patientenüberwachungszentrale abgesetzt wird.

Dort wird der Notruf vom Mobiltelefon 11 empfangen, und der Datenstring wird dem Zentralrechner 12 zugeführt und von diesem an einen PC-Arbeitsplatz 13 übergeben, wo durch einen diensthabenden Kardiologen eine sofortige Auswertung zur Analyse des Notfalls und zur Festlegung von Sofortmaßnahmen erfolgen kann.

Parallel zur Datenauswertung läuft die Ermittlung des Aufenthaltsortes des Notfallpatienten aufgrund der Anmeldung seines Mobiltelefons 7 bzw. 700 bei einer bzw. mehreren der Basisstationen 8.1 bis 8.3 des GSM-Netzes 1B sowie einer Auswertung der im System ermittelten Signallaufzeit- und ggfs. Antennenzuordnungsdaten. Aus dem Speicher 10a bei der OMC 10 werden die geografischen Daten der verbindungsaktiven Basisstation sowie derjenigen zu dieser benachbarten BTC, in Bezug auf die Signallauffzeit-Meßwerte vorliegen, in den Koordinaten-Pufferspeicher 13 ausgelesen. Die verfügbaren Laufzeitdaten - ggfs. in ihrer Zeitabhängigkeit über einen vorbestimmten Zeitraum - werden in den Laufzeit-Pufferspeicher 14 übernommen. Die Daten, die repräsentieren, über welche der Richtantennen der Basisstation(en) die Verbindung bzw. die Laufzeitmessungen erfolgt sind, werden in den Diversity-Speicher 15 geladen. Unter Zugriff auch die Inhalte dieser Speicher berechnet der Zentralrechner 12 nach bekannten Navigationsalgorithmen die Position des Mobiltelefons 7 des Notfallpatienten.

Falls die Auswertung der übermittelten Daten die Notwendigkeit eines Rettungseinsatzes ergeben hat, werden die Einsatzkräfte 14 mit der Positionsangabe und dem Auswertungsergebnis ausgestattet und können den Patienten aufsuchen.

Im übrigen kann, wenn ein Schrittmacherfehler oder etwa ein vom Schrittmacher nicht selbsttätig handhabbarer Tachykardieanfall vorliegt; über die Mobilfunkstrecke 11-7 sowie (bei der Ausführung gemäß Fig. 2) die bidirektionale Telemetriestrecke 701 a-210b ein Pragrammierungs-Eingriff am Schrittmacher vorgenommen werden, um die bedrohliche Situation ggfs. ohne Rettungseinsatz zu beenden. In diesem Zusammenhang ist es zweckmäßig, die Anordnung mit Interrogativkomponenten auszuführen, um den Erfolg der Programmierung prüfen zu können. Derartige Komponenten sind dem Fachmann bekannt und wurden daher beim vorliegenden Beispiel zugunsten der besseren Übersichtlichkeit weggelassen.

Die einmal aufgebaute Verbindung zwischen dem Patienten-Endgerät und der Überwachungszentrale kann notfalls aufrechterhalten werden, bis die Rettungskräfte beim Patienten sind. Dies ist insbesondere angezeigt, wenn die Positionsdaten eine andauernde Positionsänderung zeigen - z.B. weil ein Patient (etwa im Auto) einen aufgetretenen, potentiell bedrohlichen Schrittmacherfehler und die Aktivierung seines Mobiltelefons noch nicht bemerkt hat oder weil er sich in einem öffentlichen Verkehrsmittel befindet, das er nicht ohne weiteres anhalten kann. Anderenfalls kann die Verbindung zur Kapazitäts- und Kosteneinsparung nach Einleitung des Rettungseinsatzes von seiten der Überwachungszentrale wieder unterbrochen werden. Jedenfalls ist unter diesem Gesichtspunkt der (u.U. längerfristigen) Positionsverfolgung der Einsatz von ausreichend dimensionierten und vorzugsweise nach dem FIFO-Prinzip organisierten Pufferspeichern 13, 14 sinnvoll.

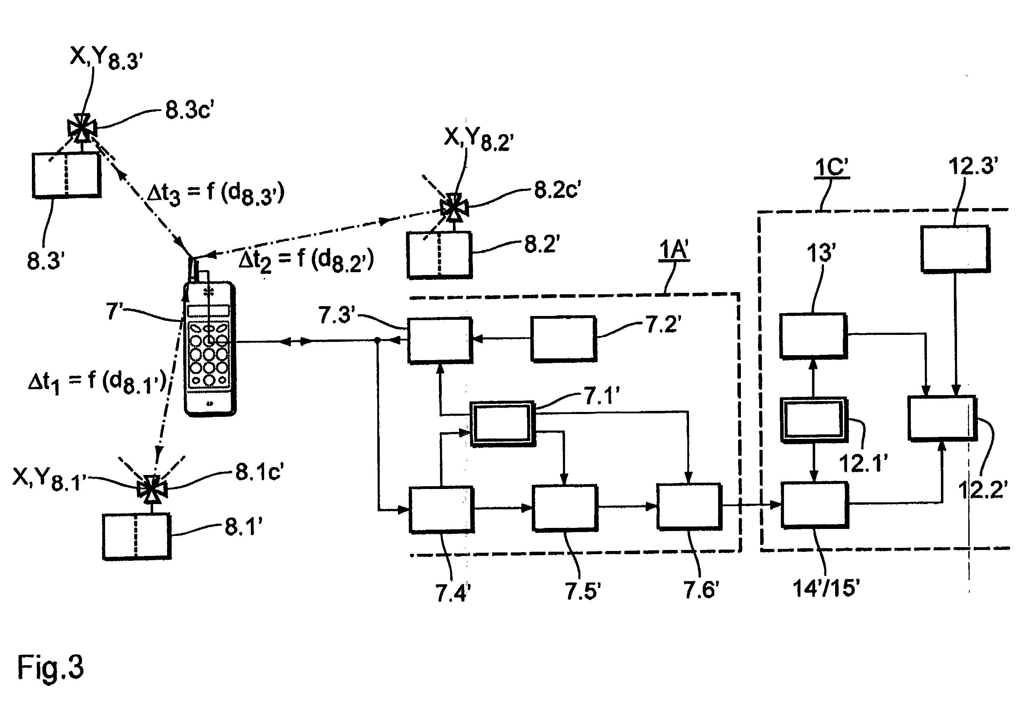

Fig. 3 zeigt in einer Ausschnittsdarstellung (wiederum als vereinfachtes Funktions-Blockschaltbild) die Mittel zur Positionsbestimmung bei einer gegenüber Fig. 1 modifizierten Ausführungsform. Die Bezugsziffern von Komponenten, die bereits in Fig.1 gezeigt sind, sind an diese angelehnt. Die Modifikation besteht vorwiegend darin, daß das Patientengerät 1 A' zur Ausführung von Laufzeitmessungen bezüglich mehrerer Basisstationen 8.1' bis 8.3' und zur Übermittlung der Laufzeitdaten an die Patientenüberwachungszentrale 1 C' ausgebildet ist.

Das Patientengerät 1 A' umfaßt hierzu eine Meßsteuereinheit 7.1', einen Meßsignalgenerator 7.2', eine über einen Steuereingang mit der Meßsteuereinheit 7.1' verbundene Meß-Ausgabeeinheit 7.3', eine Antwortsignal-Aufbereitungseinheit 7.4', eine eingangsseitig mit dieser und über einen Steuereingang mit der Meßsteuereinheit 7.1' verbundene Laufzeitberechnungseinheit 7.5' und einen mit deren Ausgang sowie ebenfalls mit der Meßsteuereinheit 7.1' verbundenen Meßergebnisspeicher 7.6'.

Bei einer Aktivierung des Patientengerätes (siehe dazu weiter oben) leitet die Meßsteuereinheit 7.1' entsprechend dem vereinbarten Betriebsprotokoll und gemäß einem gespeicherten Programm Laufzeitmessungen zu den erreichbaren Basisstationen 8.1' bis 8.3' ein, die zum Patientengerät Abstände von d_{8.1'}, d_{8.2'} bzw. d_{8.3'} haben. Dabei wird durch den Meßsignalgenerator 7.2' jeweils eine Meß- und Anfrageimpulsfolge bereitgestellt und durch die Meß-Ausgabeeinheit 7.3' unter Steuerung durch die Meßsteuereinheit 7.1' an das (nicht dargestellte) Sendeteil des Mobiltelefons 7' übergeben. Die von dessen (ebenfalls nicht dargestelltem) Empfangsteil empfangenen Antwortsignale der erreichbaren Basisstationen umfassen protokollgemäß jeweils ein Rücksignal und eine Stations- und Antennenkennung der empfangednen Antenne der jeweiligen Antennengruppe 8.1 c', 8.2c' bzw. 8.3c' und gelangen zur Antwortsignal-Aufbereitungseinheit 7.4', wo die Meßsignalkomponente separiert und der Laufzeitberechnungseinheit 7.5' zugeführt wird, während der Kennungs-String an die Meßsteuereinheit 7.1' übergeben wird. Die in der Stufe 7.5' berechneten Laufzeiten Δt₁ = f(d_{8.1'}) , Δt₂ = f(d_{8.2'}) und Δt₃ = f(d_{8.3'}) werden schließlich in Zuordnung zur zugehörigen Stations- und Antennenkennung im Meßergebnisspeicher 7.6' abgelegt.

Die Patientenüberwachungszentrale 1C' hat einen Koordinaten-Pufferspeicher 13' und einen Meßergebnis-Pufferspeicher 14'/15', der hier bei Vorliegen der Meßergebnisse aus dem Meßergebnisspeicher 7.6' des Patientengerätes 1A' gespeist wird. Eine Positionsauswertungssteuerung 12.1' greift auf die Speicher 13' und 14'/15' zu und initiiert eine Positionsberechnung in einer Berechnungseinheit 12.2' nach bekannten trigonometrischen Verfahren, deren Algorithmus in einem Auswertungsprogrammspeicher 12.3' abgelegt ist.

Hierbei werden bei Vorliegen von Laufzeitwerten zu mehreren Basisstationen aus den Laufzeiten die Abstände zu diesen und unter Heranziehung der geografischen Ortsdaten der Basisstationen der geografische Ort des Endgerätes 7' zum Zeitpunkt der Messung ermittelt. Liegen weniger als drei Laufzeitwerte vor, werden zur Erhöhung der Genauigkeit der Ortsbestimmung die gespeicherten Antennenzuordnungsdaten, die den Winkelbereich der jeweiligen Basisstation kennzeichnen, in dem sich das Patientengerät befindet, zur Auswertung mit herangezogen.

In einer aufwendigeren Ausführung sind die Meßsteuereinheit 7.1' des Patientengerätes sowie der Meßergebnis-Pufferspeicher 14'/15', die Positionsauswertungssteuerung 12.1' und die Berechnungseinheit 12.2' der Zentrale zur Ausführung bzw. Auswertung mehrerer Messungen über einen vorbestimmten Zeitraum zur Ermittlung einer Bewegungsbahn des Patientengerätes ausgebildet, die eine Positionsvorhersage zu einem zukünftigen Zeitpunkt ermöglicht. Mit einer solchen Ausführung wird ein Einsatz von Rettungskräften auch für die oben erwähnten seltenen Fälle ermöglicht, in denen der Patient im Notfall nicht ortsfest ist.

Die Erfindung beschränkt sich in ihrer Ausführung nicht auf die vorstehend erläuterten bevorzugten Ausführungsbeispiele. Vielmehr ist eine Vielzahl von Varianten denkbar, welche von der dargestellten Lösung auch in anders gearteten Ausführungen Gebrauch macht.

Die als Beispiel beschriebene Anordnung, bei der eine Auswertung der Signallaufzeiten erfolgt, kann mit im wesentlichen demselben Aufbau auch zur Auswertung der Signalfeldstärken im Mobilfunknetz ausgebildet sein. Die Auswertungsmittel sind dann bevorzugt zur Erfassung und Auswertung der (von der Signalfeldstärke der jeweiligen Basisstation abhängigen) Bitfehlerraten ausgeführt.

Das Patientengerät kann insbesondere auch als Defibrillator bzw. Kardioverter oder Medikamenteninfusionspumpe ausgeführt sein.
Bei einer ausschließlich der Zustandsüberwachung eines Patienten dienenden Anordnung ohne aktive Therapiefunktion kann anstelle einer bidirektionalen Telemetriestrecke eine unidirektionale Übertragungsstrecke zur Übertragung der relevanten Körperdaten aus dem Gerät heraus ausreichend sein.

Anstelle der "klassischen" Telemetrie können grundsätzlich auch andere kurzreichweitige Übertragungsverfahren eingesetzt werden, wobei natürlich auf einen geringen Energieverbrauch auf seiten des implantierten Teils zu achten ist.

Die oben genannten Funktionskomponenten können entsprechend den aktuellen Standards und Übertragsungsprotokollen als Hardware- oder Softwareelemente ausgeführt und funktionell in modifizierter Weise kombiniert bzw. integriert sein.

Der in der obigen Erläuterung vorausgesetzte Sprachkanal kann bei einer vereinfachten und weitgehend miniaturisierten Ausführung der Anordnung ggfs. auch entfallen.

Am Patientengerät, insbesondere am Mobilfunk-Endgerät selbst, können Alarmierungsmittel und/oder eine Anzeige relevanter Zustandsinformationen für den Patienten vorgesehen sein, die diesen hinreichend über das aufgetretene bedrohliche Problem informieren, so daß er sich ggfs. an der Lösung beteiligen kann - etwa durch sofortige Einnahme bestimmter Medikamente oder Aufsuchen einer nahegelegenen Klinik. Zur Realisierung einer entsprechenden Anzeige dient ein Umsetzer, der einen vorbestimmten Abschnitt des telemetrierten Datenstrings, mindestens den die Herkunft des Aktivierungssignals charakterisierenden Abschnitt, in eine Textinformation umsetzt.

## Patentansprüche

1. Anordnung (1) zur Patientenüberwachung, mit mindestens einem Körperfühler (2a) zur Erfassung eines physiologischen Parameters sowie einer diesem nachgeschalteten Körpersignal-Verarbeitungseinheit (3; 204, 206) und/oder einem zur Einwirkung auf den Patienten (P) ausgebildeten Therapiegerät (2; 2') und einem zur Übertragung von Daten von der Körpersignal-Verarbeitungseinheit oder dem Therapiegerät zu einer Überwachungszentrale (1C; 1C') und/oder von der Überwachungszentrale zu dem Therapiegerät ausgebildeten Mobilfunk-Endgerät (7; 700; 7'), das in einem zellulären Mobilfunknetz (1B) betreibbar ist, welches eine Mehrzahl von erdgebundenen Basisstationen (8.1 bis 8.3; 8.1' bis 8.3') aufweist, **gekennzeichnet durch** eine Basisstationskoordinaten-Speichereinheit (13; 13') und eine mit dieser verbundene Positionsbestimmungseinheit (12; 12.1' bis 12.3') zur Bestimmung des Aufenthaltsortes des Patienten aufgrund einer aus der aktuellen Basisstationsverbindung des Mobilfunk-Endgerätes in dem Mobilfunknetz gewonnenen Positionsinformation.

2. Anordnung nach Anspruch 1, **dadurch gekennzeichnet, daß** die Basistationskoordinaten-Speichereinheit (13) zum Empfang von Positionsdaten des Mobilfunk-Endgerätes (7) mit einer Mobilfunk-Vermittlungsstelle (10) verbunden ist.

3. Anordnung nach Anspruch 2, **dadurch gekennzeichnet, daß** der Überwachungszentrale (1C) Laufzeitdaten-Auswertungsmittel (12; 12.1' bis 12.3') oder Signalfeldstärke-Auswertungsmittel zur Auswertung von die Entfernung des Mobilfunk-Endgerätes (7; 7') zu mindestens einer Basisstation (8.1 bis 8.3; 8.1' bis 8.3') widerspiegelnden Laufzeitdaten bzw. Signalfeldstärkedaten zusammen mit Koordinatendaten mindestens einer aktiven Basisstation des Mobilfunknetzes (1B) zugeordnet sind.

4. Anordnung nach Anspruch 3, **dadurch gekennzeichnet, daß** die Laufzeitdaten-Auswertungsmittel (12; 12.1' bis 12.3') oder Signalfeldstärke-Auswertungsmittel zur Auswertung von Koordinatendaten und auf das Mobilfunk-Endgerät (7; 700; 7') bezogenen Laufzeit- oder Signalfeldstärkedaten mehrerer benachbarter Basisstationen (8.1 bis 8.3; 8.1' bis 8.3') aufgrund eines eine Mehrfach-Laufzeit- oder Signalfeldstärkemessung vorsehenden Betriebsprotokolls des Mobilfunknetzes (1 B) ausgebildet sind.

5. Anordnung nach einem der vorhergehenden Ansprüche, **dadurch gekennzeichnet, daß** das Mobilfunk-Endgerät (7') Mittel (7.1' bis 7.5') zur Messung der Signallaufzeit oder -feldstärke bezüglich mindestens einer Basisstation (8.1' bis 8.3') aufweist.

6. Anordnung nach einem der Ansprüche 3 bis 5, **dadurch gekennzeichnet, daß** den Laufzeitdaten-oder Signalfeldstärke-Auswertungsmitteln (12)Speichermittel (14; 14'/15') zur Speicherung der Laufzeit- bzw. Signalfeldstärkedaten, insbesondere ihrer Zeitabhängigkeit über einen vorbestimmten Zeitraum, zugeordnet sind.

7. Anordnung nach einem der Ansprüche 3 bis 6, **dadurch gekennzeichnet, daß** den Laufzeitdaten- oder Signalfeldstärke-Auswertungsmitteln (12; 12.1' bis 12.3') Antennenzuordnungs-Speichermittel (15; 14'/15') zur Speicherung der Antennenzuordnung bei der Verbindung des Mobilfunk-Endgerätes (7; 7') mit einer aktiven Basisstation (8.1 bis 8.3; 8.1' bis 8.3') zugeordnet und sie zur Auswertung von gespeicherten Antennenzuordnungsdaten mit gegenüber Laufzeit- bzw. Signlafeldstärkedaten nachrangiger Priorität ausgebildet sind.

8. Anordnung nach einem der Anspruche 3 bis 7, **dadurch gekennzeichnet, daß** die Signalfeldstärke-Auswertungsmittel Mittel zur Erfassung und Auswertung der Signal-Bitfehlerrate aufweisen.

9. Anordnung nach einem der vorhergehenden Ansprüche, **dadurch gekennzeichnet, daß** das Mobilfunknetz ein GSM-Netz (1B) ist.

10. Anordnung nach einem der vorhergehenden Ansprüche, **gekennzeichnet durch** eine mit der Körpersignal-Verarbeitungseinheit (3; 204, 206) und/oder dem Therapiegerät (2; 2') verbundene Parameterüberwachungseinheit (4; 207 bis 209, 211 bis 219) zur Meßwertüberwachung des physiologischen Parameters und/oder eines Betriebsparameters des Therapiegerätes und mit dem Ausgang der Parameterüberwachungseinheit verbundene Steuermittel (5; 705) zur Aktivierung des Mobilfunk-Endgerätes (7; 700) und Wahl einer vorprogrammierten Rufnummer im Ansprechen auf die Erfassung eines anormalen Wertes.

11. Anordnung nach einem der vorhergehenden Ansprüche, **gekennzeichnet durch** manuell betätigte Schaltmittel (7a; 706) zur Aktivierung des Mobilfunk-Endgerätes (7; 700) und Wahl einer vorprogrammierten Rufnummer.

12. Anordnung nach einem der vorhergehenden Ansprüche, **dadurch gekennzeichnet, daß** das Therapiegerät ein implantiertes Herztherapiegerät (2; 2') mit einer angeschlossenen Reiz- bzw. Schockelektrodenanordnung (2a) ist und der Körperfühler und die nachgeschaltete Körpersignal-Verarbeitungseinheit (3; 204, 206) zur Aufnahme und Verarbeitung von Herzsignalen zur Erfassung einer, insbesondere lebensbedrohlichen, Herzrhythmusstörung ausgebildet sind.

13. Anordnung nach Anspruch 10 oder 11 und 12, **dadurch gekennzeichnet, daß** die Körpersignal-Verarbeitungseinheit (3; 204, 206) eine Ratenbestimmungs- und -auswertungseinheit (206) zur Bestimmung und insbesondere auch retrospektiven Auswertung der Herzrate als physiologischem Parameter und die Parameterüberwachungseinheit Speicher- und Vergleichermittel (208, 209) zur Speicherung mindestens eines auf die Herzrate bzw. deren Zeitabhängigkeit bezogenen Grenzwertes und zum Vergleich mit diesem Grenzwert zur Ausgabe eines Steuersignals an die Steuermittel (705) bei Überschreitung des Grenzwertes aufweist.

14. Anordnung nach Anspruch 10 oder 11 und 12, **dadurch gekennzeichnet, daß** die Parameterüberwachungseinheit Erfassungs-, Speicher- und Vergleichermittel (211 bis 219) zur Überwachung mindestens der Batteriespannung und/oder der Impedanz der Reiz- bzw. Schockelektrodenanordnung und/oder der Reizimpulsamplitude zur Ausgabe eines Steuersignals an die Steuermittel (705) bei Unterschreitung eines Batteriespannungs-Grenzwertes bzw. bei Überschreitung eines Elektrodenimpedanz-Grenzwertes aufweist.

15. Anordnung nach einem der vorhergehenden Ansprüche, **dadurch gekennzeichnet, daß** dem Mobilfunk-Endgerät (7; 700) eine Schnittstelleneinrichtung (6; 704) zur Verbindung mit der Körpersignal-Verarbeitungseinheit (3; 204, 206) und/oder dem Therapiegerät (2; 2') zugeordnet ist, die Codierungsmittel (704a) zur Umsetzung von Körpersignal- bzw. Therapiegerätedaten in das Datenformat des Mobilfunknetzes (1B) aufweist.

## Claims

1. A patient monitoring arrangement (1) comprising at least one body sensor (2a) for detecting a physiological parameter and a body signal processing unit (3; 204, 206) connected downstream of the body sensor and/or a therapy device (2; 2') adapted to act on the patient (P) and a mobile telephone terminal (7; 700; 7') which is adapted to transmit data from the body signal processing unit or the therapy device to a monitoring centre (1C; 1C') and/or from the monitoring centre to the therapy device and which is operable in a cellular mobile telephone network (1B) having a plurality of ground base stations (8.1 to 8.3; 8.1' to 8.3'), **characterised by** a base station co-ordinate storage unit (13; 13') and a position determining unit (12; 12.1' to 12.3') connected thereto for determining the location of the patient on the basis of an item of positional information obtained from the current base station connection of the mobile telephone terminal in the mobile network.

2. An arrangement according to claim 1 **characterised in that** the base station co-ordinate storage unit (13) is connected to a mobile telephone exchange (10) for receiving positional data of the mobile telephone terminal (7).

3. An arrangement according to claim 2 **characterised in that** associated with the monitoring centre (1C) are transit time data evaluation means (12; 12.1' to 12.3') or signal field strength evaluation means for evaluating transit time data or signal field strength data respectively reflecting the distance of the mobile telephone terminal (7; 7') in relation to at least one base station (8.1 to 8.3; 8.1' to 8.3') together with coordinates of at least one active base station of the mobile telephone network (1B).

4. An arrangement according to claim 3 **characterised in that** the transit time data evaluation means (12; 12.1' to 12.3') or signal field strength evaluation data are adapted for the evaluation of co-ordinate data and transit time or signal field strength data related to the mobile telephone terminal (7; 700; 7') of a plurality of adjacent base stations (8.1 to 8.3; 8.1' to 8.3') on the basis of an operating protocol of the mobile telephone network (1B), which provides a multiple transit time or signal field strength measuring procedure.

5. An arrangement according to one of the preceding claims **characterised in that** the mobile terminal (7') has means (7.1' to 7.5') for measuring the signal transit time or field strength with respect to at least one base station (8.1' to 8.3').

6. An arrangement according to one of claims 3 to 5 **characterised in that** associated with the transit time data or signal field strength evaluation means (12) are storage means (14; 14'/15') for storage of the transit time or signal field strength data, in particular the time dependency thereof over a predetermined period of time.

7. An arrangement according to one of claims 3 to 6 **characterised in that** associated with the transit time data or signal field strength evaluation means (12; 12.1' to 12.3') are antenna association storage means (15; 14'/15') for storage of the antenna association upon connection of the mobile telephone terminal (7; 7') to an active base station (8.1 to 8.3; 8.1' to 8.3') and they are adapted to evaluate stored antenna association data with a priority of lower rank than transit time or signal field strength data respectively.

8. An arrangement according to one of claims 3 to 7 **characterised in that** the signal field strength evaluation means have means for detecting and evaluating the signal bit error rate.

9. An arrangement according to one of the preceding claims **characterised in that** the mobile telephone network is a GSM network (1B).

10. An arrangement according to one of the preceding claims **characterised by** a parameter monitoring unit (4; 207 to 209, 211 to 219) connected to the body signal processing unit (3; 204, 206) and/or the therapy unit (2; 2'), for measurement value monitoring of the physiological parameter and/or an operating parameter of the therapy device and control means (5; 705) connected to the output of the parameter monitoring unit for activation of the mobile telephone terminal (7; 700) and dialling of a pre-programmed call number in response to the detection of an abnormal value.

11. An arrangement according to one of the preceding claims **characterised by** manually actuated switching means (7a; 706) for activation of the mobile telephone terminal (7; 700) and dialling of a pre-programmed call number.

12. An arrangement according to one of the preceding claims **characterised in that** the therapy device is an implanted cardiac therapy device (2; 2') with a connected stimulus or shock electrode arrangement (2a) and the body sensor and the body signal processing unit (3; 204, 206) connected downstream thereof are adapted to pick up and process cardiac signals for the detection of an in particular life-threatening cardiac arrhythmia.

13. An arrangement according to claim 10 or claim 11 and claim 12 **characterised in that** the body signal processing unit (3; 204, 206) has a rate determining and evaluation unit (206) for determining and in particular also retrospectively evaluating the heart rate as a physiological parameter and the parameter monitoring unit has storage and comparator means (208, 209) for the storage of at least one limit value related to the heart rate or the time dependency thereof and for comparison with said limit value for the output of a control signal to the control means (705) when the limit value is exceeded.

14. An arrangement according to claim 10 and claim 11 and claim 12 **characterised in that** the parameter monitoring unit has detection, storage and comparator means (211 to 219) for monitoring at least the battery voltage and/or the impedance of the stimulus or shock electrode arrangement and/or the stimulus pulse amplitude for the output of a control signal to the control means (705) when the battery voltage falls below a battery voltage limit value or when an electrode impedance limit value is exceeded.

15. An arrangement according to one of the preceding claims **characterised in that** associated with the mobile telephone terminal (7; 700) is an interface device (6; 704) for connection to the body signal processing unit (3; 204, 206) and/or the therapy device (2; 2'), which has encoding means (704a) for conversion of body signal or therapy device data into the data format of the mobile telephone network (1B).

## Revendications

1. Système (1) de surveillance du patient, comportant au moins un capteur physiologique (2a) destiné à enregistrer un paramètre physiologique, ainsi qu'une unité de traitement des signaux physiologiques (3 ; 204, 206) montée en aval de celui-ci et/ou un appareil thérapeutique (2; 2'), conçu pour influer sur le patient (P), et un terminal de téléphonie mobile (7; 700; 7'), qui est conçu pour la transmission de données à partir de l'unité de traitement des signaux physiologiques ou à partir de l'appareil thérapeutique vers une centrale de surveillance (1C; 1C') et/ou à partir de la centrale de surveillance vers l'appareil thérapeutique et qui peut être utilisé dans un réseau de radiotéléphonie mobile cellulaire (1B) qui comporte une pluralité de postes de base (8.1 à 8.3; 8.1' à 8.3') reliés à la terre, **caractérisé par** une unité de mémoire des coordonnées des postes de base (13; 13') et une unité de localisation (12; 12.1' à 12.3') reliée à celle-ci et destinée à déterminer le lieu de séjour du patient en se basant sur une information de position obtenue à partir de la liaison actuelle du poste de base du terminal de téléphonie mobile dans le réseau de radiotéléphonie mobile.

2. Système selon la revendication 1, **caractérisé en ce que** l'unité de mémoire des coordonnées des postes de base (13) est reliée à un central de téléphonie mobile (10) en vue de recevoir les données de localisation du terminal de téléphonie mobile (7).

3. Système selon la revendication 2, **caractérisé en ce que** la centrale de surveillance (1C) est associée à des moyens d'analyse des données de temps de transfert (12; 12.1' à 12.3') ou des moyens d'analyse de l'intensité des champs de signaux, destinés à analyser, conjointement avec les données de coordonnées d'au moins un poste de base actif du réseau de radiotéléphonie mobile (1B), les données de temps de transfert ou les données d'intensité des champs de signaux, reflétant la distance entre le terminal de téléphonie mobile (7; 7') et au moins un poste de base (8.1 à 8.3; 8.1' à 8.3').

4. Système selon la revendication 3, **caractérisé en ce que** les moyens d'analyse des données de temps de transfert (12 ; 12.1' à 12.3') ou les moyens d'analyse de l'intensité des champs de signaux sont conçus pour analyser les données de coordonnées et les données de temps de transfert ou d'intensité de champs de signaux, rapportées au terminal de téléphonie mobile (7; 700; 7'), de plusieurs postes de base (8.1 à 8.3; 8.1' à 8.3') voisins en se basant sur un protocole de service, prévoyant une mesure multiple du temps de transfert ou une mesure de l'intensité des champs de signaux, du réseau de radiotéléphonie mobile (1B).

5. Système selon l'une quelconque des revendications précédentes, **caractérisé en ce que** le terminal de téléphonie mobile (7') comporte des moyens (7.1' à 7.5') destinés à mesurer le temps de transfert des signaux ou l'intensité des champs de signaux par rapport à au moins un poste de base (8.1' à 8.3').

6. Système selon l'une quelconque des revendications 3 à 5, **caractérisé en ce que** les moyens d'analyse des données de temps de transfert ou les moyens d'analyse de l'intensité des champs de signaux (12) sont associés à des moyens de mémoire (14; 14'/15') destinés à stocker les données de temps de transfert ou les données d'intensité des champs de signaux, en particulier leur dépendance avec le temps pendant une période prédéterminée.

7. Système selon l'une quelconque des revendications 3 à 6, **caractérisé en ce que** les moyens d'analyse des données de temps de transfert ou les moyens d'analyse de l'intensité des champs de signaux (12; 12.1' à 12.3') sont associés à des moyens de mémoire de l'affectation des antennes (15; 14'/15') destinés à stocker l'affectation des antennes pendant la communication entre le terminal de téléphonie mobile (7; 7') et un poste de base (8.1 à 8.3; 8.1' à 8.3') actif et ils sont conçus pour analyser les données d'affectation des antennes stockées, qui ont une priorité inférieure aux données de temps de transfert ou d'intensité des champs de signaux.

8. Système selon l'une quelconque des revendications 3 à 7, **caractérisé en ce que** les moyens d'analyse de l'intensité des champs de signaux comportent des moyens destinés à enregistrer et analyser les taux d'erreurs sur les bits des signaux.

9. Système selon l'une quelconque des revendications précédentes, **caractérisé en ce que** le réseau de radiotéléphonie mobile est un réseau GSM (1B).

10. Système selon l'une quelconque des revendications précédentes, **caractérisé par** une unité de surveillance des paramètres (4 ; 207 à 209 ; 211 à 219), reliée à l'unité de traitement des signaux physiologiques (3 ; 204, 206) et/ou à l'appareil thérapeutique (2 ; 2') et destinée à surveiller la valeur de mesure du paramètre physiologique et/ou d'un paramètre de service de l'appareil thérapeutique, et des moyens de commande (5 ; 705) reliés à la sortie de l'unité de surveillance des paramètres et destinés, en réponse à la détection d'une valeur anormale, à activer le terminal de téléphonie mobile (7 ; 700) et à sélectionner un numéro d'appel préprogrammé.

11. Système selon l'une quelconque des revendications précédentes, **caractérisé par** des moyens de commutation (7a, 706), actionnés manuellement et destinés à activer le terminal de téléphonie mobile (7 ; 700) et à sélectionner un numéro d'appel préprogrammé.

12. Système selon l'une quelconque des revendications précédentes, **caractérisé en ce que** l'appareil thérapeutique est un appareil cardiologique (2 ; 2') implanté, auquel est raccordé un système d'électrodes de stimulation ou de chocs (2a), et le capteur physiologique et l'unité de traitement des signaux physiologiques (3 ; 204, 206), montée en aval, sont conçus pour recevoir et traiter les signaux cardiologiques en vue de détecter des troubles du rythme cardiaque, en particulier mettant en danger la vie.

13. Système selon la revendication 10 ou les revendications 11 et 12, **caractérisé en ce que** l'unité de traitement des signaux physiologiques (3 ; 204, 206) comporte une unité de détermination et d'analyse des taux (206) pour la détermination et, en particulier aussi, pour l'analyse rétrospective du taux cardiaque en tant que paramètre physiologique, et l'unité de surveillance des paramètres comporte des moyens de mémoire et de comparaison (208, 209) pour stocker au moins une valeur seuil relative au taux cardiaque ou à sa dépendance avec le temps et pour effectuer une comparaison avec cette valeur seuil en vue d'émettre un signal de commande vers les moyens de commande (705) en cas de dépassement de la valeur seuil.

14. Système selon la revendication 10 ou les revendications 11 et 12, **caractérisé en ce que** l'unité de surveillance des paramètres comporte des moyens d'enregistrement, de mémoire et de comparaison (211 à 219) destinés à surveiller au moins la tension de la batterie et/ou l'impédance du système d'électrodes de stimulation ou de chocs et/ou l'amplitude de l'impulsion de stimulation, en vue d'émettre un signal de commande vers les moyens de commande (705) en cas de dépassement de la valeur seuil de la tension de la batterie ou en cas de dépassement de la valeur seuil de l'impédance du système d'électrodes.

15. Système selon l'une quelconque des revendications précédentes, **caractérisé en ce que** le terminal de téléphonie mobile (7; 700) est associé à une unité d'interface (6; 704), qui est destinée à établir la liaison avec l'unité de traitement des signaux physiologiques (3; 204, 206) et/ou l'appareil thérapeutique (2; 2') et qui comporte des moyens de codage (704a) destinés à convertir les données des signaux physiologiques et de l'appareil thérapeutique dans le format des données du réseau de radiotéléphonie mobile (1B).
